# EUROPEAN PATENT APPLICATION

(11) **EP 3 300 733 A1**
(43) Date of publication of application: **04.04.2018**
(21) Application number: 16306275.5
(22) Date of filing: 30.09.2016
(51) Int. Cl.: A61K 31/4745, A61K 31/513, A61P 21/00

(54) **SUBSTANCE SELECTED AMONG IRINOTECAN, AN IRINOTECAN ACTIVE METABOLITE, 5-FLUORO-URACILE, A PHARMACEUTICAL SALT THEREOF AND A MIXTURE THEREOF, FOR USE IN THE TREATMENT OF FIBROSIS**

(71) Applicant: Centre Hospitalier Universitaire de Bordeaux, 33404 Talence (FR); Université de Bordeaux, 33000 Bordeaux (FR)
(72) Inventor: BONNET, Fabrice, 33000 BORDEAUX (FR)
(74) Representative: Novagraaf Technologies

(57) **Abstract**

The present invention relates to a substance selected from the group comprising irinotecan, an irinotecan active metabolite, 5-fluoro-uracile, a pharmaceutical salt thereof and a mixture thereof, for use in the treatment of fibrosis.

## Description

### Technical field

The present invention refers to a substance for use in the treatment of fibrosis.

Therefore, the present invention has utility in the medical and pharmaceutical fields.

In the description below, the references into brackets ([ ]) refer to the listing of references situated at the end of the text.

### Background of the Invention

Scleroderma (systemic sclerosis) is a complex disease in which extensive fibrosis, vascular alterations, and autoantibodies against various cellular antigens are among the principal features (Gabrielli A. et al. Scleroderma. N Engl J Med. 2009; 360: 1989-2003 ([1**])).** There are two major subgroups in the commonly accepted classification of scleroderma: limited cutaneous scleroderma and diffuse cutaneous scleroderma.

In limited cutaneous scleroderma, fibrosis is mainly restricted to the hands, arms, and face. Raynaud's phenomenon is present for several years before fibrosis appears, pulmonary hypertension is frequent, and anti-centromere antibodies occur in 50 to 90% of patients.

Diffuse cutaneous scleroderma is a rapidly progressing disorder that affects a large area of the skin and compromises one or more internal organs. Diffuse cutaneous scleroderma is mostly associated with anti-topoisomerase-I (topo-I) antibodies that represent the most frequent antibody associated with systemic scleroderma.

In cases of limited cutaneous scleroderma, the prognosis remains quite good (overall survival at 10 years: 80-90%). Nevertheless, the occurrence of hypertension (10%) or of lung fibrosis (20 to 30%) is of bad prognosis.

In cases of diffuse cutaneous scleroderma, the prognosis is more severe with an overall survival at 10 years of 60-80% because of the high risk of lung fibrosis (30 to 56%), pulmonary arterial hypertension (12 to 27%), heart involvement (10 to 32%), scleroderma renal crisis (7 to 17%).

The available data indicate a scleroderma prevalence of 50 to 300 cases per million persons with an incidence ranging from 2.3 to 22.8 cases per million persons per year. It represents around 6,500 to 10,000 persons in France (Chifflot H. et al.: "Incidence and prevalence of systemic sclerosis". Semin Arthritis Rheum 2008; 37: 223-235 ([2**])).** Women are at higher risk of scleroderma with a ratio ranging from 3:1 to 14:1 in the different epidemiologic studies.
The cause of scleroderma is still unknown **([1])**. The pathogenesis of scleroderma includes microvascular damage, mononuclear-cell infiltrate and fibrosis.

Vascular injury is an early event in scleroderma. It precedes fibrosis and involves small vessels, particularly the arterioles. The vascular damage, which occurs in virtually all organs, consists of large gaps between endothelial cells, loss of integrity of the endothelial lining, and vacuolization of endothelial-cell cytoplasm. In addition, there are several basal lamina-like layers, perivascular infiltrates of mononuclear immune cells (with rare lymphocytes) in the vessel wall, obliterative microvascular lesions, and rarefaction of capillaries. The remarkable paucity of small blood vessels is a characteristic found in later stages of scleroderma.

Fibrosis gradually replaces the vascular inflammatory phase of scleroderma and ultimately disrupts the architecture of the affected tissue. It is the cause of the main symptoms of the disease. Fibrosis in the skin begins in the lower dermis and upper subcutaneous layer and occurs together with loss of microvasculature, reduction of appendages, and loss of reticular structure and the rete ridges. The composition of accumulated matrix varies with the stage of the disease. A mixture of different collagen types, proteoglycans, and elastic fibers including fibrillin is typical of the early stages, whereas type I collagen accumulates in later stages.

Fibroblasts appear to orchestrate the production, deposition, and remodeling of collagens and other extracellular-matrix components. Fibroblasts in scleroderma are heterogeneous in terms of collagen synthesis. Overproduction of collagen is due to enhanced transcription or increased stability of collagen-specific messenger RNA. Upregulated transcription of collagen genes in scleroderma cells is autonomous and maintained in vitro over several passages.

Scleroderma is associated with several autoantibodies, some of which are important diagnostic markers. Tests for autoantibodies against topo-I (Scl-70), centromere-associated proteins, and nucleolar antigens can be useful in facilitating the diagnosis and formulating a prognosis. Although the autoantibodies correlate with disease severity and the risk of specific organ complications, their pathogenic relevance is unclear.

Anti-topo-I antibodies are the most frequent autoantibodies during diffuse scleroderma. They are associated with severe systemic and visceral involvement of scleroderma (pulmonary fibrosis, cardiac, digestive and joint involvement, scleroderma renal crisis).

Human DNA topo-I is a 765-amino-acid nuclear enzyme involved in topological changes of DNA structure. It plays key roles in DNA replication, transcription, and recombination.

Pathogenic role of anti-topo-I antibodies in cellular and animal model is not proven and they could only represent a marker of immune and inflammatory activity along diffuse scleroderma.

However, some animals and in vitro studies have shown the following findings:
- Treatment with topo-I and adjuvant induce skin and lung fibrosis and autoimmune abnormalities in mice (Yoshizaki A, Yanaba K, Ogawa A, Asano Y, Kadono T, Sato S. Immunization with DNA Topoisomerase I and Freund's complete adjuvant induce skin and lung fibrosis and autoimmunity via IL-6 signaling. Arthritis Rheum 2011; 63: 3575-85 ([3**])).** Treatment with topo-I and adjuvant also induces anti-topoisomerase antibodies and Th2 and Th17 cell lymphocytes activation in bronchoalveolar lavage fluid.
- The topo-I specific T cells can become clonally expanded in some patients and may contribute to the pathogenesis (Hu PQ, Oppenheim JJ, Medsger Jr. TA, Wright TM. T cell lines from systemic sclerosis patients and healthy controls recognize multiple epitopes on DNA topoisomerase I. J Autoim 2006; 26: 258-67 ([4**])).**
- Topo-I binding to fibroblast surfaces is both necessary and sufficient for anti-topo-I binding. Topo-I-anti-topo-I complex binding can then trigger the adhesion and activation of monocytes, thus providing a plausible model for the amplification of the fibrogenic cascade in anti-topo-Ipositive SSc patients (Henault G. et al.DNA Topoisomerase I binding to fibroblasts induces monocyte adhesion and activation in the presence of anti-topoisomerase I autoantibodies from systemic sclerosis patients. Arthritis Rheum 2006; 54 : 963-73 ([5**])).**
- Selective oxidation of DNA topo-I induces systemic sclerosis in the mouse and is associated with anti-topo-I antibodies occurrence, endothelial activation and fibroblast proliferation (Servettaz A, Goulvestre C, Kavian N, et al. Selective oxidation of DNA topoisomerase 1 induces systemic sclerosis in the mouse. J Immunol 2009; 182: 5855-64 ([6**])).** These results suggest that oxidized topo-I is not only the target of immune response but also a main contributor of oxidative stress and organic lesions.
- Topo-I stimulated the phosphorylation of several phospholipase intracellular signaling pathways that stimulated fibroblast migration. Topo-I was also found to interact directly with CCR7, a potent leukocyte chemotactic receptor, playing an important role in the initiation of the immune response, and was later described as a regulator of the homing of immune cells, such as T cells and dendritic cells, toward secondary lymphoid organs (Arcand J, Robitaille G, Koenig M, Senecal JL, Raymond Y. The Autoantigen DNA topoisomerase I interacts with chemokine receptor 7 and exerts cytokine-like effects on dermal fibroblasts. Arthritis Rheum 2012; 64: 826-34 ([7**])).**

In the context of SSc pathophysiology, fibroblast opsonization followed by the binding of topo-I/anti-topo-I complexes leads to the adhesion and activation of monocytes. These cells could further lead to the initiation and maintenance of an inflammatory cascade, stimulating the fibrosis that is characteristic of SSc-associated with anti-topo-I.

There is no specific therapeutic for scleroderma. The aims of the treatment are to limit immune inflammation and fibrosis. Corticosteroids are often used in the field of auto-immune diseases but they are not efficient in scleroderma. On the opposite they are involved in an increased risk of scleroderma renal crisis. They are usually used at low dosage (< 10 mg per day) in rheumatologic involvement.

The uses of immunosuppressive agents are frequent in case of extensive skin or visceral involvement (lung, heart, kidney, digestive tract). The most frequent immunosuppressive agents used in scleroderma are cyclophosphamide, azathioprine, methotrexate but they are poorly effective on skin and visceral manifestations of scleroderma.

Because of the poor results of these treatments, an international consortium implemented a therapeutic trial evaluating autologous hematopoietic stem cell transplantation vs intravenous pulse cyclophosphamide in diffuse cutaneous systemic sclerosis (van Laar JM, Farge D, Sont JK et al. Autologous hematopoietic stem cell transplantation vs intravenous pulse cyclophosphamide in diffuse cutaneous systemic sclerosis: a randomized clinical trial. JAMA 2014; 311: 2490-8. ([8**])).**

A total of 156 patients were randomly assigned to receive HSCT (Hematopoietic stem cell transplantation) (n = 79) or cyclophosphamide (n = 77). During a median follow-up of 5.8 years, 53 events occurred: 22 in the HSCT group (19 deaths and 3 irreversible organ failures) and 31 in the control group (23 deaths and 8 irreversible organ failures). During the first year, there were more events in the HSCT group (13 events [16.5%], including 8 treatment-related deaths) than in the control group (8 events [10.4%], with no treatment-related deaths). At 2 years, 14 events (17.7%) had occurred cumulatively in the HSCT group vs 14 events (18.2%) in the control group; at 4 years, 15 events (19%) had occurred cumulatively in the HSCT group vs 20 events (26%) in the control group.

In conclusion, patients in the HCST group experienced higher mortality in the first year but had better long-term overall survival than those treated with cyclophosphamide alone. However, Grade 3 or 4 adverse events occurred in 51 patients (62.9%) in the HSCT group and 30 (37.0%) in the control group (P = .002). Viral infections were detected in 22 patients (27.8%) in the HSCT group vs 1 (1.3%) in the control group (P < 0.001).

This recent trial underlines the high level of mortality and of morbidity of diffuse scleroderma, whatever the immunosuppressive treatment in a selected population.

Among immunosuppressive agents, rituximab has recently shown interesting results in case series and in a case-control study (Jordan S, Distler JHW, Maurer B, et al. Effects and safety of rituximab in systemic sclerosis: an analysis from the European Scleroderma Trial and Research (EUSTAR) group. Ann Rheum Dis Online First, published on January 17, 2014 as 10.1136/annrheumdis-2013-204522 ([9**]))** and is being evaluated in a French randomized study versus placebo in joint involvement of systemic scleroderma (the Recover trial).
A phase two, randomized, controlled study failed to demonstrate efficacy of tocilizumab, an interleukin 6 receptor-α inhibitor, on the reduction in skin thickening in adults with progressive systemic sclerosis (Khanna D. et al. al. Safety and efficacy of subcutaneous tocilizumab in adults with systemic sclerosis (faSScinate): a phase 2, randomised, controlled trial. Lancet. 2016; 387: 2630-40 ([10**])).**

Others therapeutic alternatives are symptomatic and include endothelin receptor antagonism like bosentan and sildenafil. These treatments are indicated in case of pulmonary hypertension or digital chronic ulcer but have no effect on the fibrosis of scleroderma (Launay D, Sitbon O, Le Pavec J, et al. Long-term outcome of systemic sclerosis-associated pulmonary arterial hypertension treated with bosentan as first-line monotherapy followed or not by the addition of prostanoids or sildenafil. Rheumatology (Oxford). 2010; 49: 490-500 ([11**])).** Many other "antifibrotic" agents have been evaluated in open series (colchicine, calcitriol, halofunginone) but none of them has be found to be effective on the excessive production of collagen by the fibroblast.

Pulmonary fibrosis represents one of the main complications of systemic scleroderma. Pulmonary fibrosis may also be idiopathic and is also a disease with a poor prognosis. It affects five millions people worldwide (Kekevian A. et al.: "Diagnosis and classification of idiopathic pulmonary fibrosis." Autoimmun Rev 2014;13: 508-512 ([12**])).** The pathogenesis of idiopathic pulmonary fibrosis is largely unknown but results anyway to a profibrotic state with findings of fibroblast proliferation resulting in excessive secretion of collagen and other proteins. These thickened and fibrotic area cause damage to lung architecture and decrease gas exchanges (Fletcher S, Jones GJ, Spinks, K, et al. The safety of new drugs treatment for idiopathic pulmonary fibrosis; Expert Opin Drug Saf 2016; 17: 1-7 ([13**])).**

Two drugs have been approved for clinical use in idiopathic pulmonary fibrosis (Canestaro WJ et al.: "Drug Treatment of Idiopathic Pulmonary Fibrosis: Systematic Review and Network Meta-Analysis." Chest 2016; 149: 756-66 ([14**])).** Pirfenidone, an oral pyridine has combined anti-oxydant, anti-inflammatory, and anti-fibrotic actions in experimental models although the mechanism of action remains unknown. However, it has been shown in a 72 week phase III trial to reduce decline in lung function of only 4.4% (forced vital capacity, FCV) on one year, and another trial failed to demonstrate significant activity on lung fibrosis progression. Nintedanib is a tyrosine kinase inhibitor that suppresses signaling receptors implicated in the pathogenesis of fibrosis. In two randomized trials, it has been shown to significantly reduce lung function progression compared to placebo at one year **([14]).** However, neither of these treatments has been shown to have a benefit on overall and respiratory mortality. To note, these two drugs have not been tested in scleroderma and one previous trial failed to demonstrated any efficacy of tyrosine kinase inhibitors in the field of scleroderma (Prey S et al. Imatinib mesylate in scleroderma-associated diffuse skin fibrosis: a phase II multicentre randomized double-blinded controlled trial. Br J Dermatol 2012; 167: 1138-44 ([15**])).**

Thus, a need exists of alternative treatments of fibrosis, in particular scleroderma. The present invention fulfills these and other needs.

### Description of the invention

The Applicant has found surprisingly that irinotecan or an irinotecan active metabolite and 5-fluoro-uracile (5FU), or a pharmaceutically acceptable salt thereof, or a prodrug thereof, when administered alone or in combination together, can efficiently treat fibrosis, and in particular sclerodermic skin involvement in patients with scleroderma.

Indeed, surprisingly, the results obtained by the Applicant lead to the conclusion that anti-topo-I autoantibodies present in the sera of patients with systemic sclerosis (SSc) could exert their pathophysiologic effects by interfering with the normal role of topo-I as a danger signal for fibroblasts.

All together, these findings represent arguments for a key role of topo-I in the pathogenesis of scleroderma since topo-I is overexpressed in scleroderma and is able to activate monocytes, T-lymphocytes and fibroblasts, and to induce fibrosis and anti-topo-I autoantibodies.

Thanks to the results obtained by the Applicant, it may be hypothesized that irinotecan, an irinotecan active metabolite and/or 5-FU may act as an anti-topoisomerase on topoisomerase enzyme that is overexpressed in scleroderma, in particular in diffuse scleroderma or through an anti-fibrotic mechanism.

In addition to anti-fibrotic affects, the results obtained by the Applicant lead surprisingly to the conclusion that immunomodulation of B-cell activity could complete the efficacy of irinotecan or an active metabolite thereof and/or 5FU on scleroderma by analogy to the benefit observed with rituximab treatment.

At last, irinotecan or an active metabolite thereof and/or 5-FU may have an impact on the vascular injury of scleroderma, an early event in the pathogenic process of scleroderma, by decreasing inflammation at this early stage of the disease.

Advantageously, irinotecan, an irinotecan active metabolite, 5FU, or any of their combination, may have a high and specific activity on collagen production by fibroblasts of sclerodermic skin, and may allow remission of this disease.

Combination of irinotecan and 5FU is currently used in the treatment of cancers, but its efficacy in scleroderma was, until the present invention, totally unknown.

Accordingly, in a first aspect, the present invention provides a substance selected from the group comprising irinotecan, an irinotecan active metabolite, 5-fluoro-uracile, a pharmaceutical salt thereof, a prodrug thereof, a combination thereof and a mixture thereof, for use in the treatment of fibrosis.

"Irinotecan" refers herein to a semisynthetic analogue of the natural alkaloid camptothecin having the following formula (I):

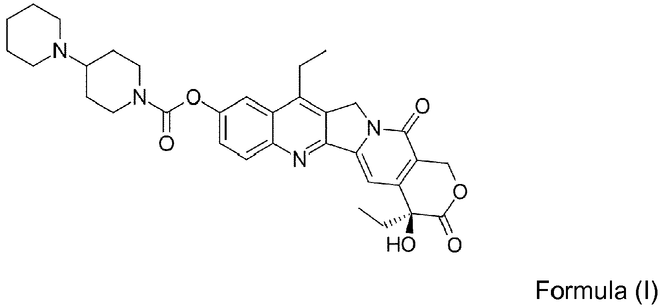

It may be designated under its chemical name (S)-4,11-diethyl-3,4,12,14-tetrahydro-4-hydroxy-3,14-dioxo1H-pyrano[3',4':6,7]-indolizino[1,2-b]quinolin-9-yl-[1,4'bipiperidine]-1'-carboxylate. Its CAS number is 97682-44-5. Irinotecan is activated by hydrolysis to SN-38, an active metabolite which is, as irinotecan, an inhibitor of topoisomerase I. This is then inactivated by glucuronidation by uridine diphosphate glucoronosyltransferase 1A1 (UGT1A1). The inhibition of topoisomerase I by irinotecan or the active metabolite SN-38 eventually leads to inhibition of both DNA replication and transcription. Irinotecan is currently used as Camptosar^{™} or Campto^{™} or as a generic of irinotecan hydrochloride trihydrate for the treatment of cancer.

"Irinotecan active metabolite" refers herein to any therapeutically active metabolite released by activation of irinotecan, for example within the human body by an enzymatic hydrolysis. It may advantageously refer to SN-38, which is the active metabolite of irinotecan. An active metabolite of irinotecan may have at least the same degree of biological activity as irinotecan, or may be more active than irinotecan, for example about 1.5 or 2 or 3 or 4 or 50 or 100 or 1000 time more active.

SN-38 has the following formula (III):

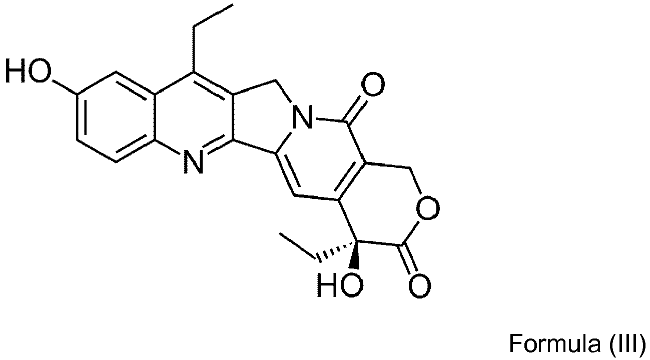

SN-38 may be designated under its chemical name 7-Ethyl-10-hydroxy-camptothecin. Its CAS number is 86639-52-3.

"5-fluoro-uracile" refers herein to a pyrimidine analog having the following formula (III):

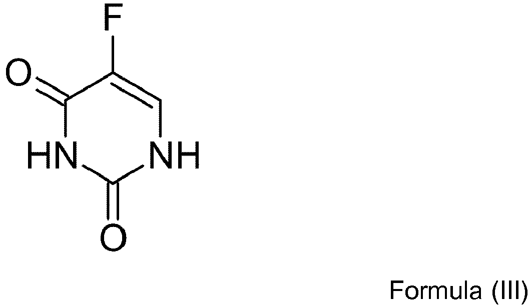

It may be designated under its chemical name 5-Fluoro-1H,3H- pyrimidine-2,4-dione. Its CAS number is 51-21-8. 5-FU and it is currently used in the treatment of cancer under its trade name Adrucil™. It is antimetabolite and works through irreversible inhibition of thymidylate synthase. Thymidylate synthase normally methylates deoxyuridine monophosphate (dUMP) to form thymidine monophosphate (dTMP). Administration of 5-FU causes a scarcity in dTMP, so rapidly dividing cancerous cells which undergo cell death via thymineless death. Calcium folinate provides an exogenous source of reduced folinates and hence stabilises the 5-FU-TS complex, hence enhancing 5-FU's cytotoxicity.

The term "pharmaceutical salt" is meant to include any pharmaceutically acceptable salt, the substance that are prepared with relatively nontoxic acids or bases, depending on the particular substituents found on the compounds described herein. The pharmaceutical salts include any salt suitable to be administered to humans or animals. Examples of non toxic pharmaceutically acceptable salts may include hydrochloride, carboxylate sodium, carboxylate disodium, sodium, sulfuric acid, nitric acid, phosphoric acid, methanesulfonic acid, citric acid, maleic acid, and succinic acid. Pharmaceutically acceptable salt of irinotecan may be preferably hydrochloride, for example Camptosar®, or carboxylate sodium. Pharmaceutically acceptable salt of SN-38 may be preferably carboxylate disodium salt. Pharmaceutically acceptable salt of 5FU may be preferably a sodium salt.

"Prodrug" refers herein to a medication or compound that, after administration, is metabolized in the body to a pharmacologically active drug. It may be for example an amide form of 5FU.

"Mixture" refers herein to any association comprising irinotecan and 5FU, or an association comprising an irinotecan active metabolite and 5FU, for example an association comprising SN-38 and 5FU, or anyone of their pharmaceutical salt. Advantageously, these associations may have a synergistic effect on the inhibition of the production of collagen by scleroderma fibroblasts and may represent a specific treatment for scleroderma, especially diffuse scleroderma.

Advantageously, the mixture may comprise oxaliplatine. So, the mixture may further be understood as an association comprising irinotecan and oxaliplatine, or 5FU and oxaliplatine, or an association comprising an irinotecan active metabolite and oxaliplatine, for example an association comprising SN-38 and oxaliplatine, or an association comprising irinotecan, oxaliplatine and 5FU, or an association comprising an irinotecan active metabolite, oxaliplatine and 5FU, or anyone of their pharmaceutical salt.

"Combination" refers herein to an administration of irinotecan and 5FU, or an administration of an irinotecan active metabolite and 5FU, for example an administration of SN-38 and 5FU, or anyone of their pharmaceutical salt, in which the compounds are physically separated, and that may be realized simultaneously, separately or sequentially. Examples of sequential administration is FOLFORINOX and FOLFOX protocols.

"Fibrosis" refers herein to the pathological accumulation of extracellular matrix (ECM) proteins, leading to the formation of excess fibrous connective tissue, including collagen and glycosaminoglycans, in an organ or tissue in a reparative or reactive process. Fibrosis may include scleroderma (systemic sclerosis), notably limited cutaneous scleroderma and diffuse cutaneous scleroderma, pulmonary fibrosis due to a scleroderma, idiopathic pulmonary fibrosis, cystic fibrosis, hepatic fibrosis and cirrhosis, atrial fibrosis, endomyocardial fibrosis, myocardial infarction, glial scar, arthrofibrosis, fibrotic manifestation of graft versus host disease, fibrosis associated with IgG4 related diseases, radiation fibrosis, Crohn's Disease, Dupuytren's contracture, keloid, mediastinal fibrosis, myelofibrosis, nephrogenic systemic fibrosis, progressive massive fibrosis and retroperitoneal fibrosis. In some cases, fibrosis may be associated with topoisomerase autoantibodies, especially Topo I autoantibodies. Preferably, the fibrosis is a scleroderma, notably limited cutaneous scleroderma or diffuse cutaneous scleroderma, preferably diffuse cutaneous scleroderma. Alternatively, the fibrosis may be in particular an idiopathic pulmonary fibrosis and/or retroperitoneal fibrosis and/or cystic fibrosis.

"Treatment" refers herein to any improvement of at least one symptom of the fibrosis, or to the recovery of the fibrosis, or to the prevention of the symptoms of the fibrosis, and especially the early vascular phase of the disease. The recovery of the fibrosis may be a decrease or a disappearance of the fibrosis. The at least one symptom that may be improved may be selected among the formation of excess fibrous connective tissue and/or collagen overproduction and/or accumulation of extracellular matrix (ECM) proteins. In addition, when fibrosis is scleroderma, especially diffuse cutaneous scleroderma, symptom that may be improved may be selected among skin involvement, a decrease of the thickness and hardness of the skin at any localization, an improvement of the mouth shrinking, a decrease of face telangiectasia, an improvement of sclerodactyly, an esthetic improvement of the skin, an improvement of the Rodnan score and/or an improvement of visceral injury of scleroderma (lung, heart, digestive tract, kidney, joints, or nerves). The improvement of at least one symptom may refer to total or partial disappearance of at least one symptom, which may be transient or definitive.

Advantageously, the substance may decrease the production of collagen by fibroblasts implied in fibrosis, especially of fibroblasts of sclerodermic skin.

Advantageously, the substance may decrease the vascular inflammation at the early stage of the fibrosis, especially in scleroderma.

Advantageously, the substance may act as an immunomodulatory agent in the fibrosis, especially in fibrosis associated with autoimmune diseases, especially in scleroderma.

Advantageously, the substance may decrease the expression level of genes involved in the production of collagen by fibroblasts implied in fibrosis, especially of fibroblasts of sclerodermic skin.

Advantageously, the substance may decrease the level of topoisomerase autoantibody in the subject.

Advantageously, the substance may be administered to a patient in need thereof to a pharmaceutically acceptable and efficient dose for the treatment of fibrosis.

For example, the substance may be administrated once per week, or once per two weeks, or once per three weeks, or once per month.

For example, one dose of irinotecan or its active metabolite may be from 1 to 700mg/m², for example from 20 to 700 mg/m² or from 50 to 500mg/m² and particularly 350 mg/m² intravenously over 90 minutes every three weeks when it is administered as a single agent. Alternatively, one dose of irinotecan may be from 20 to 350 mg/m² when administered with 5FU and/or oxaliplatine, for example 180 mg/m² intravenously over 90 minutes every other week for three doses. The dose may be administered over 45 min, or over 60 minutes, or over 90, or other 120 minutes.

For example, 5FU may be administered at a dose of from 100 to 700 mg/m² every week or every three weeks or every four weeks, for example from 3 to 6 days per month when it is administered as a single agent. Alternatively, a dose may be from 40 to 150 mg/m²/day, administered from 2 to 5 days, spaced out of 3 to 4 weeks when 5FU is administered with irinotecan, an irinotecan active metabolite or oxaliplatine.

For example, oxaliplatine may be administered at a dose of 85 mg/m² intravenously every 2 weeks, for 6 months.

According to the invention, the substance may be administered for sufficient time for the treatment of fibrosis as defined above. In this purpose, the substance may be administered for one month, or for two months, or for three months, or for six months, or for at least six months, or for the patient's lifetime.

Administration may be carried out directly, i.e. pure or substantially pure, or after mixing of the substance with a pharmaceutically acceptable carrier and/or medium.

Administration of the substance may be carried out either simultaneously, separately or sequentially with at least one another active compound(s) selected in the group comprising corticosteroids, immunosuppressive and biologic agents as cyclophosphamide, azathioprine, methotrexate, hematopoietic stem cell transplantation, rituximab, tocilizumab, riociguat, endothelin receptor antagonism like bosentan or sildenafil, prostacyclin analog like iloprost or epoprostenol or treprostinil, phosphodiesterase type 5 inhibitors sildenafil, antifibrotic agents as colchicine, calcitriol, halofunginone, pirfenidone, nintedanib. The substance and the at least one another active compound(s) mixture may be administered in a relative amount of 0:1 to 1:0, for example 1:1. The other active compound may be used for the treatment of fibrosis, or for another pathology existing together with the fibrosis, for example arthralgia, arthritis, gastro-oesophageal reflux disease and other digestive injuries, renal diseases, lung disease for example pulmonary hypertension or cutaneous ulcers.

In one embodiment, the administration is a parenteral administration. The parenteral administration may be selected from the group comprising intravenous administration, intramuscular administration and subcutaneous administration. In those cases, the substance may be in the form of an injectable solution.

In another embodiment, the administration may be an oral administration. In this embodiment, it may be a buccal or a sublingual administration. It may for example be in the form selected from the group comprising a liquid formulation, an oral effervescent dosage form, an oral powder, a pill, a multiparticule system, an orodispersible dosage form, a solution, a syrup, a suspension, an emulsion and oral drops. When the medicament is in the form of an oral effervescent dosage form, it may be in a form selected from the group comprising tablets, granules, powders. When the medicament is the form of an oral powder or a multiparticulate system, it may be in a form selected from the group comprising beads, granules, mini tablets and micro granules. When the medicament is the form of an orodispersible dosage form, it may be in a form selected from the group comprising orodispersible tablets, lyophilized wafers, thin films, a chewable tablet, a tablet and a capsule, a medical chewing gum. According to the present invention, when the medicament is for buccal and sublingual routes, it may be selected from the group comprising buccal or sublingual tablets, muco adhesive preparation, oro-mucosal drops and sprays.

In another embodiment, the administration may be a transdermal or a transmucosal administration. When the medicament is for topical-transdermal administration, it may be selected from the group comprising ointments, cream, gel, lotion, patch and foam. It may also be a medicament for nasal administration, for example selected from the group comprising nasal drops, nasal spray, nasal powder.

In another embodiment, the administration may be a rectal administration, for example suppository or hard gelatin capsule. The skilled person in the art understands clearly that the term "form" as used herein refers to the pharmaceutical formulation, including veterinary formulation, of the medicament for its practical use. For example, the medicament may be in a form selected from the group comprising an injectable form (for example as in Folforinox protocol), oral suspension (for example as Efferalgan^{®}3%), a pellet (for example as Dafalgan^{®}1g), powder (for example as Doliprane^{®}100mg ), granules (for example as Zoltum^{®}10mg ), spray, transdermal patch (for example as Cordipatch^{®}5mg/24h ) or local form (cream, lotion, collyrium) (for example as Dermoval creme^{®}, as Betneval^{®}lotion and as Chibroxine^{®} collyre respectively). In these examples, the substance may be added or may replace the active ingredient(s) of said medicaments.

The pharmaceutically acceptable carrier may be any known suitable pharmaceutically and/or veterinary carrier used for the administration of a substance to a human or to an animal, depending on the subject. For example, this carrier may be one or more carrier(s) selected from the group comprising for example the monomethoxy-polyethyleneglycol (for example as in Viraferonpeg^{®}), Liposome (for example as in Ambizome^{®}), magnesium stearate (E572), talc (E553b), Silicon dioxide (E551), microcrystalline cellulose (E460) and maize starch. Preferably, the carrier comprises magnesium stearate (E572), talc (E553b), Silicon dioxide (E551), microcrystalline cellulose (E460) and maize starch.

The medium may be any known medium used for the administration of a substance to a human or to an animal. For example, this medium may be selected from the group comprising for example cremophor (for example as in Sandimmun^{®}) or cellulosis (for example as in Avlocardyl^{®} LP160mg).

The pharmaceutical form of the drug is selected with regard to the human or animal to be treated. For example, for a child or a baby, a syrup or an injection is preferred. Administration may be carried out with a weight graduated pipette.

In a second aspect, the present invention provides a method of treating a subject suffering from a fibrosis as defined above, comprising a step of administering to said subject a substance as defined above.

In another aspect, the present invention provides the use of a substance as defined above for the manufacture of a medicament for the treatment of a fibrosis as defined above.

This invention is further illustrated by the following examples with regard to the annexed drawings that should not be construed as limiting.

### Brief description of the figures

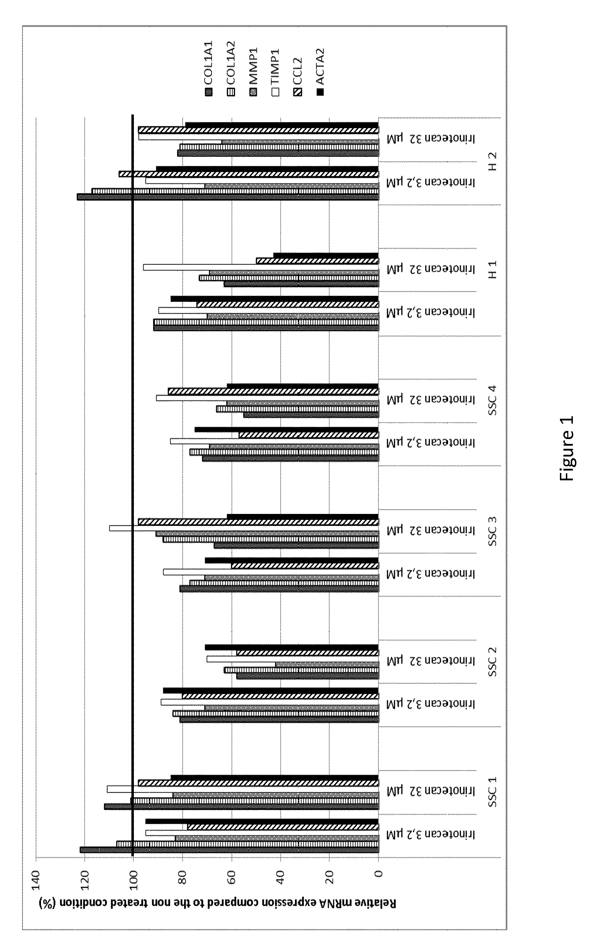

- Figure 1: represents the relative expression of, from left to right per panel, COL1A1, COL1A2, MMP1, TIMP1, CCL2 and ACTA2 mRNA in dermal fibroblasts obtained from 4 systemic sclerosis patients (SSC 1 to SSC4) or 2 healthy patients (H1, H2) treated with irinotecan (3.2 or 32 µM), in comparison to a non-treated condition. The non-treated condition was arbitrary fixed at 100%. A level above 100% corresponds to a gene overexpression in the treated condition and a level below 100% corresponds to a gene downregulation in the treated condition.

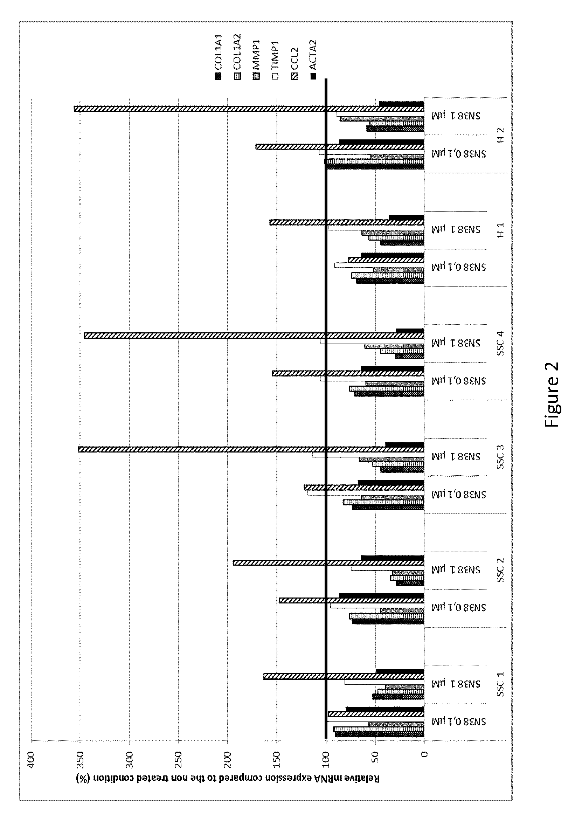

- Figure 2: represents the relative expression of, from left to right per panel, COL1A1, COL1A2, MMP1, TIMP1, CCL2 and ACTA2 mRNA in dermal fibroblasts obtained from 4 systemic sclerosis patients (SSC 1 to SSC4) or 2 healthy patients (H1, H2) treated with SN38 (0.1 or 1 µM) in comparison to a non-treated condition. The non-treated condition was arbitrary fixed at 100%. A level above 100% corresponds to a gene overexpression in the treated condition and a level below 100% corresponds to a gene downregulation in the treated condition.

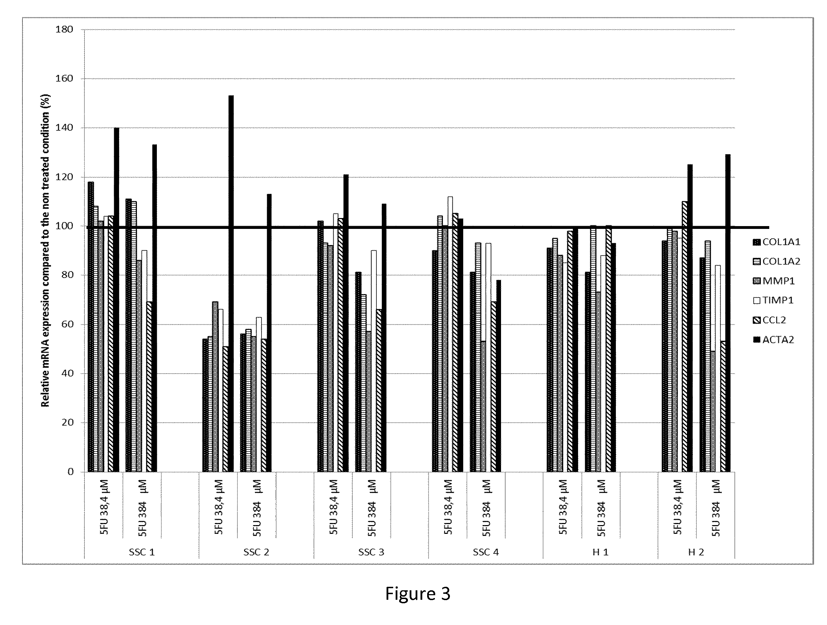

- Figure 3: represents the relative expression of, from left to right per panel, COL1A1, COL1A2, MMP1, TIMP1, CCL2 and ACTA2 mRNA in dermal fibroblasts obtained from 4 systemic sclerosis patients (SSC 1 to SSC4) or 2 healthy patients (H1, H2) treated with 5FU (38.4 or 384 µM) in comparison to a non-treated condition. The non-treated condition was arbitrary fixed at 100%. A level above 100% corresponds to a gene overexpression in the treated condition and a level below 100% corresponds to a gene downregulation in the treated condition.

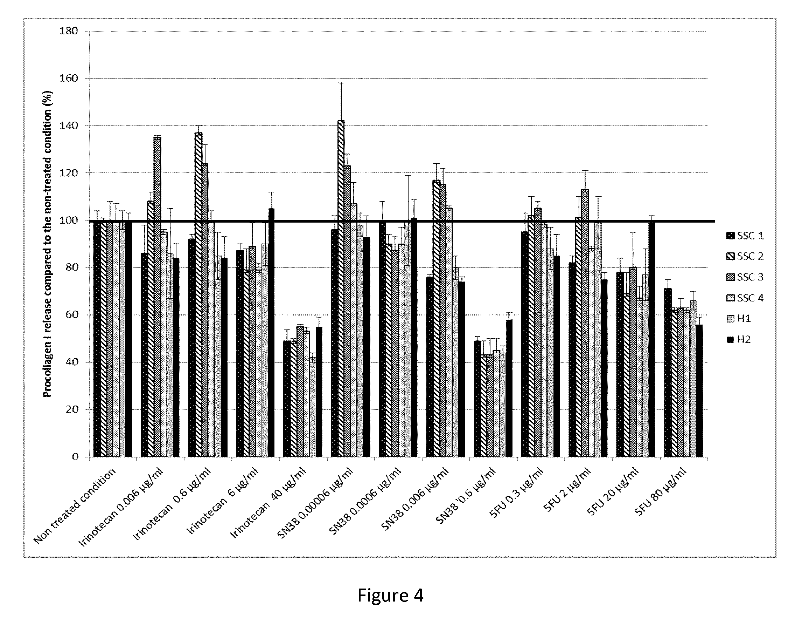

- Figure 4: represents the treatment effect on procollagen I release. The level of procollagen I release was evaluated in fibroblasts from (from left to right per panel) 4 SSC patients (SSC1-SSC4) or from 2 healthy donors (H1-H2). A comparison of procollagen I release level was done between non-treated condition and treated conditions. 4 different doses of irinotecan (i.e. 0.006 µg/ml, 0.6 µg/ml, 6 µg/ml, 40 µg/ml), SN38 (i.e. 0.00006 µg/ml, 0.0006 µg/ml, 0.006 µg/ml, 0.6 µg/ml) or 5FU (i.e. 0.3 µg/ml, 2 µg/ml, 20 µg/ml, 80 µg/ml) were evaluated.

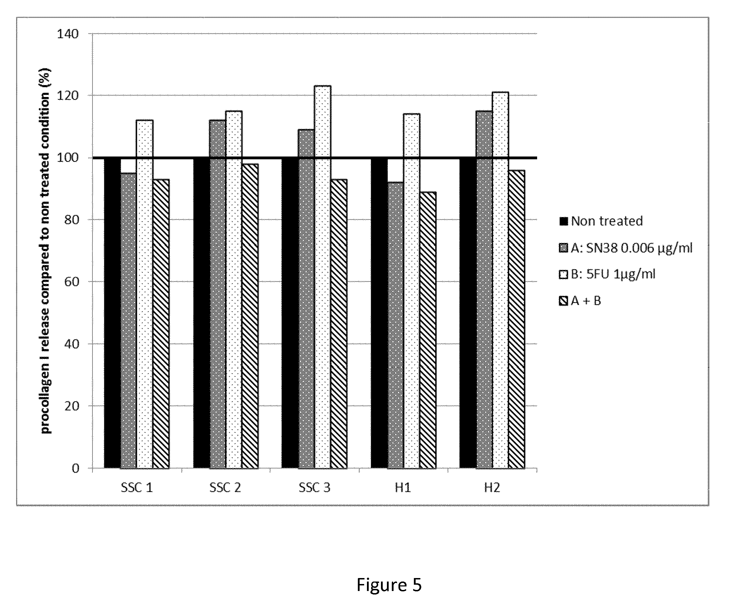

- Figure 5: represents the treatment effect on procollagen I release. The level of procollagen I release was evaluated in fibroblasts from 3 SSC patients (SSC1-SSC3) or from healthy donors (H1-H2). A comparison of procollagen I release level was done between non-treated (100%) condition and treated condition. Slight doses of SN38 (0.006 µg/mL) and 5FU (1 µg/mL) were evaluated alone or in combination.

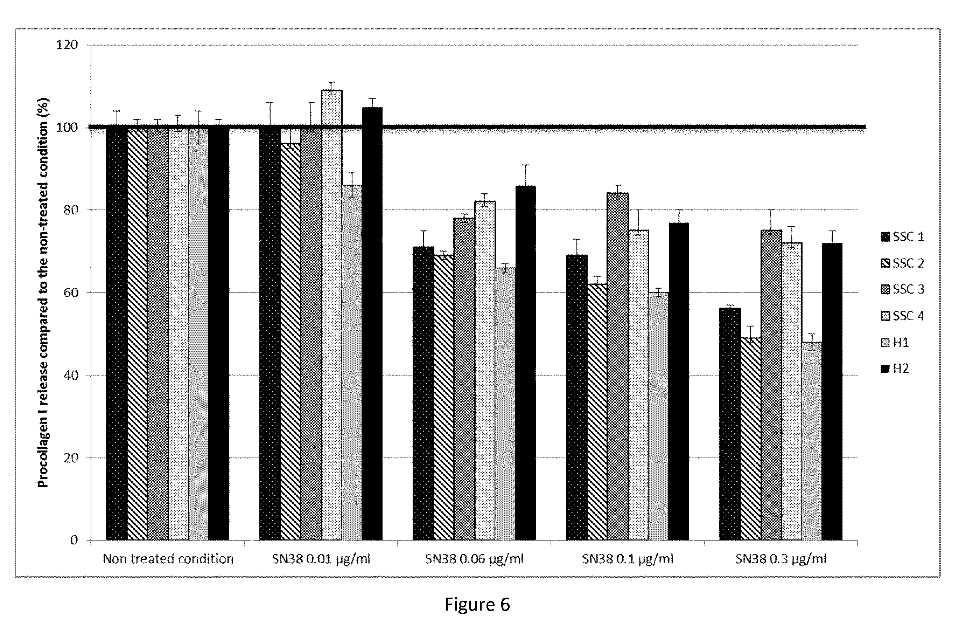

- Figure 6: represents the treatment effect on procollagen I release. The level of procollagen I release was evaluated in fibroblasts from (from left to right per panel) 4 SSC patients (SSC1-SSC4) or from 2 healthy donors (H1-H2). A comparison of procollagen I release level was done between non-treated condition and treated conditions. 4 different doses of SN38 (i.e. 0.01 µg/ml, 0.06 µg/ml, 0.1 µg/ml, 0.3 µg/ml) were evaluated.

### Examples

### Example 1: In vitro studies of the effect of subcytotoxic doses irinotecan, SN38, 5FU

### 1. Evaluation of the in vitro effect of subcytotoxic doses of irinotecan on mRNA expression of a panel of fibrosis genes

Fibrosis associated to systemic sclerosis occurs when inappropriate tissue remodeling results in excess extracellular matrix (ECM) deposition. The effect of irinotecan was assessed on a panel of 6 key genes involved in extracellular matrix remodeling: COL1A1 and COL1A2 which are ECM components, MMP1 and TIMP1 which are remodeling enzymes, CCL2 which is a pro-fibrotic gene coding for an inflammatory chemokine and ACTA2 (alpha-SMA) which is a pro-fibrotic gene. The effect of irinotecan was assessed in 4 pairs of SSC (SSC1 to SSC4) and 2 pairs of healthy control (H1 or H2) dermal fibroblasts (Figure 1). The two concentrations (3.2 µM and 32 µM) of irinotecan used are not cytotoxic on the fibroblasts (data not shown). Despite a slight variability observed between the fibroblasts batches, the treatment with irinotecan led to a global downregulation of genes involved in fibrosis. The highest evaluated dose of irinotecan resulted in an inhibition of COL1A1 (average inhibition of 27 %), COL1A2 (average inhibition of 22%), MMP1 (average inhibition of 32 %), CCL2 (average inhibition of 19 %), ACTA2 (average inhibition of 33 %) mRNA levels in both SSc and healthy fibroblasts, compared from the same lot of fibroblasts (SSC1, SSC2, SSC4, SSC4, H1 or H2) in non-treated condition. For 3 patients (SSC2, SSC4, H1), a decrease was observed for all the mRNA genes.

### 2. Evaluation of the in vitro effect of subcytotoxic doses of an irinotecan active metabolite on mRNA expression of a panel of fibrosis genes

The effect of SN38 was assessed in 4 pairs of SSC and 2 pairs of healthy control (H) dermal fibroblasts. The two concentrations (0.1 µM and 1 µM) of SN38 used are not cytotoxic on the fibroblasts (data not shown). The treatment with SN38 led to a global downregulation of genes involved in fibrosis except for CCL2 gene (Figure 2). The highest evaluated dose of SN38 resulted in an important inhibition of COL1A1 (average inhibition of 57.5 %), COL1A2 (average inhibition of 52%), MMP1 (average inhibition of 42 %), TIMP1 (average inhibition of 22 %), ACTA2 (average inhibition of 55 %) mRNA levels in both SSc and healthy fibroblasts, compared from the same lot of fibroblasts (SSC1, SSC2, SSC4, SSC4, H1 or H2) in non-treated condition.

### 3. Evaluation of the in vitro effect of subcytotoxic doses of 5-fluoro-uracile on mRNA expression of a panel of fibrosis genes

The effect of 5-fluoro-uracile (5FU) was assessed in 4 pairs of SSC and 2 pairs of healthy control (H) dermal fibroblasts (Figure 3). The two concentrations (38.4 µM and 384 µM) of 5FU used are not cytotoxic on the fibroblasts (data not shown). The treatment with 5FU led to a downregulation of genes involved in fibrosis. The highest evaluated dose of 5FU resulted in an inhibition of COL1A1 (average inhibition of 17%), COL1A2 (average inhibition of 12%), MMP1 (average inhibition of 37 %), TIMP1 (average inhibition of 15 %) and CCL2 (average inhibition of 31 %) mRNA levels in both SSc and healthy fibroblasts, compared from the same lot of fibroblasts (SSC1, SSC2, SSC4, SSC4, H1 or H2) in non-treated condition.

### 4. Dose response to irinotecan, SN38 or 5FU on the release of procollagen I protein

The main manifestation of systemic sclerosis is the overproduction of extracellular matrix and especially type I collagen. The effect of irinotecan, SN38 or 5FU on the release of procollagen I protein was assessed in 4 pairs of SSC and 2 pairs of healthy control (H) dermal fibroblasts (Figure 4). Three independent experiments were done. Four concentrations of each molecule (irinotecan: 0.006 µg/ml, 0.6 µg/ml, 6 µg/ml, 40 µg/ml, SN38: 0.00006 µg/ml, 0.0006 µg/ml, 0.006 µg/ml, 0.6 µg/ml or 5FU: 0.3 µg/ml, 2 µg/ml, 20 µg/ml, 80 µg/ml) were assessed and compared to a non-treated condition. A supplementary set of 3 concentrations of SN38 (SN38: 0.01 µg/ml, 0.06 µg/ml, 0.1 µg/ml, 0.3 µg/ml) was evaluated in three independent experiments (Figure 6). All the doses used are not cytotoxic on the fibroblasts (data not shown). Incubation with 40µg/mL irinotecan, 0.3 µg/mL SN38, 0.6 µg/mL SN38 or 80µg/mL 5FU for 48h significantly decreased the release of procollagen I protein in both cell types.

### 5. Effect of a combination of slight SN38 and 5FU doses on the release of procollagen I protein

The relevance of combining very low non-cytotoxic doses of SN38 and 5FU was assessed in 3 pairs of SSC and 2 pairs of healthy control (H) dermal fibroblasts (Figure 5). The effect of a low dose of SN38 (0.006 µg/ml = condition A) or a low dose of 5FU (1 µg/ml = condition B) were compared to a non-treated condition and also to a condition combining the two low doses of SN38 and 5FU (condition A+B). Surprisingly, even if the quantitative inhibition is very slight because the doses used were very low, combining the two molecules led to an effect on the inhibition better than the one obtained with SN38 alone or 5FU alone. More precisely, for example for patient SSC2, SSC3 or H2, the combination of SN38 and 5FU led to an inhibition of the procollagen I release. Such an inhibition was not observed for SN38 alone or 5FU alone.

### 6. Materials and Methods of the in vitro studies

### Cell culture

Dermal fibroblasts were obtained from clinically affected skin of systemic sclerosis (SSC) patients and from healthy donors (H). The fibroblasts were grown in DMEM medium containing 2mM of L-glutamine supplemented with 10% foetal bovine serum (FCS) and penicillin/streptomycin antibiotics. For the experiments, cells were starved 24h before the addition of irinotecan, SN38 or 5FU in DMEM 0-1% FCS. mRNA analysis

Dermal fibroblasts were plated in DMEM 10% FCS in 24-well plates. 24h before the experiment, medium was switched to DMEM 0% FCS. Cells were treated for 24h with irinotecan (3.2µM or 32 µM), SN38 (0.1 or 1µM) or 5FU (38.4µM or 384µM) in DMEM 0% FCS. Supernatants were removed and plates were freezed at -80°c. Fibroblasts RNA from each well-plate were extracted with NucleoSpin® RNA Plus kit (Macherey-Nagel) according to manufacter's instructions. Triplicates were done and RNA from triplicates were pooled together. The quantity and quality of RNA were evaluated by capillary electrophoresis (Bioanalyzer 2100, Agilent).

RNA was used for cDNA synthesis using the Transcriptor Reverse Transcriptase kit (Roche). qPCR reactions were run on a Light Cycler (Roche Molecular Systems Inc.) according to manufacter's instructions. The relative gene expression levels of COL1A1, COL1A2, MMP1, TIMP1, CCL2 and ACTA2 were calculated using the ΔΔCt method. Target mRNA levels were normalized to GAPDH.

Expression of the genes in the different treated conditions was presented as fold change compared with the gene expression of the same non-treated fibroblasts arbitrary fixed at 100%.

Primers used are listed below in Table 1:

**Table 1:**

| **gene** | **Abbreviation** | **Gene Bank** | **primer sens** | **primer antisens** | **cDNA bp** |
|---|---|---|---|---|---|
| Collagen, type I, alpha 1 | COL1A1 | NM_000088 | CGATGGATTCCAGTTCGAGTA (SEQ ID NO. 1) | GTTTACAGGAAGCAGACAGG (SEQ ID NO. 2) | 420 |
| Collagen, type I, alpha 2 | COL1A2 | NM_000089 | AGGTGTAAGCGGTGGTGGTTATGAC (SEQ ID NO. 3) | CCGGATACAGGTTTCGCCAGTAGAG (SEQ ID NO. 4) | 313 |
| Matrix metallopeptidase 1 | MMP1 | NM_002421 | ACTGCTGCTGCTGCTGTTCTG (SEQ ID NO. 5) | TGCTTCATCACCTTCAGGGTTTCAG (SEQ ID NO. 6) | 249 |
| TIMP metallopeptidase inhibitor 1 | TIMP1 | NM_003254 | TGCAATTCCGACCTCGTCATCAGGGC (SEQ ID NO. 7) | AGAAACTCCTCGCTGCGGTTGTGGG (SEQ ID NO. 8) | 215 |
| Chemokine (C-C motif) ligand 2 | CCL2 | NM_002982 | TTCTCAAACTGAAGCTCGCACTCTCGCC (SEQ ID NO. 9) | TGTGGAGTGAGTGTTCAAGTCTTCGGAGTT (SEQ ID NO. 10) | 348 |
| Actin, alpha 2, smooth muscle, aorta | ACTA2 | NM_001613 | AATGGCTCTGGGCTCTGTAA (SEQ ID NO. 11) | TGGTGATGATGCCATGTTCT (SEQ ID NO. 12) | 199 |

### Determination of procollagen I release

Dermal fibroblasts were plated in DMEM 10% FCS supplemented with 20µg/mL of vitamin C in 96-well plates. 24h before the experiment, medium was switched to DMEM 0% FCS supplemented with 20µg/mL of vitamin C. Cells were treated for 48h with irinotecan (0.006, 0.6, 6 or 40µg/mL), SN38 (0.00006, 0.006, 0.006, 0.01, 0.06, 0.1, 0.3 or 0.6µg/mL) or 5FU (0.3, 2, 20 or 80µg/mL) in DMEM 0% FCS supplemented with 20µg/mL of vitamin C. Supernatants were collected to evaluate the procollagen I release level by ELISA kit (ref MK101, supplier Takara) according to manufacter's instructions. Triplicates were done. The level of procollagen I release was presented as fold change compared with the procollagen I release by the same non-treated fibroblasts arbitrary fixed at 100%.

### Effect of combination on procollagen I release

Dermal fibroblasts were plated in DMEM 10% FCS supplemented with 20µg/mL of vitamin C in 96-well plates. 24h before the experiment, medium was switched to DMEM 0% FCS supplemented with 20µg/mL of vitamin C. Cells were treated for 48h with SN38 (0.006 µg/mL), 5FU (1 µg/mL) or a combination (SN38 0.006 µg/mL and 5FU 1 µg/mL) in DMEM 0% FCS supplemented with 20µg/mL of vitamin C. Supernatants were collected to evaluate the procollagen I release level by ELISA kit (ref MK101, supplier Takara) according to manufacter's instructions. Triplicates were done. The level of procollagen I release was presented as fold change compared with the procollagen I release by the same non-treated fibroblasts arbitrary fixed at 100%.

### Example 2: Description of a clinical case

### Case report

A 56 years old man was referred for fever and abdominal pain. His medical history was mainly represented by a systemic scleroderma diagnosed 20 years before and with digestive, cutaneous and rheumatologic involvement. Before admission, he was treated for 5 years with methotrexate, 7.5 mg to 10 mg per week without substantial impact on scleroderma evolution. Blood test found anti topo-I antibodies to high level (141 IU/mL, normal range 0-29). Physical examination revealed the changes of diffuse scleroderma: flexion contractures of the fingers with sclerodactyly, tightness of the face, microstomia, and skin tightness at the arms, chest, and feet. The modified Rodnan score was 24/51.

Fever and abdominal pain finally revealed a rectum adenocarcinoma stage pT3 N1 Mx. Then, it was proposed to the patient 4 cycles of chemotherapy FOLFORINOX followed by rectal surgery.

The patient received 4 cycles of FOLFORINOX (oxaliplatine 85 mg/m², irinotecan 180 mg/m², 5-fluoro-uracil 400 mg/m² flash then 2400 mg/m² the following 46 hours). Cycles of chemotherapy were administered every 14 days on January 13 and 27, and February 11 and 25, 2014.

The patient described as soon as the second cycle of chemotherapy a significant improvement of cutaneous sclerosis particularly in the last region affected by the disease such as face, body and proximal region of the arms. He reported a decrease of the thickness and hardness of the skin by 20 to 30% on the shoulder and on the top of the truck, and by 30% on the face. He also described an improvement of the mouth shrinking by 50% and a decrease of face telangiectasia after the 4 cycles of chemotherapy. Forearm and hand were also improved in term of thickness, softness and arthralgia by 20%.

After 4 cycles of chemotherapy, the Rodnan score was 18 (improved by 6 points in 8 weeks). In the same time, the Mouth Handicap in Systemic Sclerosis questionnaire (MHISS) improved from 18/48 to 12/48 and the Health Assessment Questionnaire improved from 31/60 to 26/60 with a gain on walking, dressing, catching.

Then, the patient had surgery and according to post-surgical piece, it was realized 6 cycles of FOLFOX (folinic acid, oxaliplatine and 5-fluorouracil) with no new improvement during these last 6 cycles.

Twelve months later, the general status of the patients was stable and the Rodnan Score also at 18/51; 18 months later, methotrexate was restarted because the occurrence of arthralgia on the hand.

### Discussion

There is no similar observation in the literature.

One 59 year-old patient treated with topotecan for an ovarian cancer developed in the following weeks a scleroderma without anti-topo I antibodies (Ene-Stroescu D, Ellman MH, Peterson CE. Topotecan and the development of scleroderma or a scleroderma-like illness. Arthritis Rheum 2005; 43: 840-50 ([16**])).**

There is no association reported in the literature between scleroderma and oxaliplatin.

### Hypothesis

Regarding our case report , we conclude that the irinotecan (or its active metabolite) and/or 5FU may have a synergistic effect on the inhibition of the production of collagen by scleroderma fibroblasts and could represents a specific treatment for diffuse scleroderma.

### Tolerance of the association

The therapeutic association irinotecan-5FU is very well known in the field of colon cancer for more than 12 years. It is indicated in second line in patients with metastatic colon cancer or in first line, in combination with oxaliplatin in a context of neo-adjuvant treatment.

The synergy between irinotecan and 5FU has already been demonstrated in cancer models (Rami G. Azrak et al. Therapeutic Synergy Between Irinotecan and 5-fluorouracil against Human Tumor Xenografts. Clin Cancer Res 2004 ;10 :1121-9 ([17**])).**

It is also a well-tolerated combination since it is proposed to quite old patients with metastatic cancer (mostly hepatic) and with altered general conditions.

The main clinical side effects of the combination are diarrhea (22 to 44% of the patient receiving the therapeutic combination), fatigue (6 to 7%), vomiting (3 to 11%), nausea (2 to 7%), cholinergic syndrome (1 to 2%).

Neutropenia was noticed in 29 to 41% of the patients.

### Dose of the association

Recent publication regarding the interest of irinotecan in murin models of Systemic Lupus Erythematosus (SLE) have suggested that irinotecan have immune-modulatory affects at dose 50 times lower than the dose applied for chemotherapy in humans. Moreover, low-dose irinotecan reduce B-cell activity that is possibly an additional interest for this molecule.

### Reference List

1. Gabrielli A, Avvedimento EV, Krieg T. Scleroderma. N Engl J Med 2009; 360: 1989-2003.
2. Chifflot H, Fautrel B, Sordet C, Chatelus E, Sibilia J. Incidence and prevalence of systemic sclerosis. Semin Arthritis Rheum 2008; 37: 223-235.
3. Yoshizaki A, Yanaba K, Ogawa A, Asano Y, Kadono T, Sato S. Immunization with DNA Topoisomerase I and Freund's complete adjuvant induce skin and lung fibrosis and autoimmunity via IL-6 signaling. Arthritis Rheum 2011; 63: 3575-85.
4. Hu PQ, Oppenheim JJ, Medsger Jr. TA, Wright TM. T cell lines from systemic sclerosis patients and healthy controls recognize multiple epitopes on DNA topoisomerase I. J Autoim 2006 ; 26 : 258-67.
5. Henault G, Robitaille G, Senecal JL, Raymond Y. DNA Topoisomerase I binding to fibroblasts induces monocyte adhesion and activation in the presence of anti-topoisomerase I autoantibodies from systemic sclerosis patients. Arthritis Rheum 2006; 54: 963-73.
6. Servettaz A, Goulvestre C, Kavian N, et al. Selective oxidation of DNA topoisomerase 1 induces systemic sclerosis in the mouse. J Immunol 2009; 182: 5855-64.
7. Arcand J, Robitaille G, Koenig M, Senecal JL, Raymond Y. The Autoantigen DNA topoisomerase I interacts with chemokine receptor 7 and exerts cytokine-like effects on dermal fibroblasts. Arthritis Rheum 2012; 64: 826-34.
8. van Laar JM, Farge D, Sont JK et al. Autologous hematopoietic stem cell transplantation vs intravenous pulse cyclophosphamide in diffuse cutaneous systemic sclerosis: a randomized clinical trial. JAMA 2014; 311: 2490-8.
9. Jordan S, Distler JHW, Maurer B, et al. Effects and safety of rituximab in systemic sclerosis: an analysis from the European Scleroderma Trial and Research (EUSTAR) group. Ann Rheum Dis Online First, published on January 17, 2014 as 10.1136/annrheumdis-2013-204522.
10. Khanna D, Denton CP, Jahreis A, et al. Safety and efficacy of subcutaneous tocilizumab in adults with systemic sclerosis (faSScinate): a phase 2, randomised, controlled trial. Lancet. 2016; 387: 2630-40.
11. Launay D, Sitbon O, Le Pavec J, et al. Long-term outcome of systemic sclerosis-associated pulmonary arterial hypertension treated with bosentan as first-line monotherapy followed or not by the addition of prostanoids or sildenafil. Rheumatology (Oxford) 2010; 49 :490-500.
12. Kekevian A, Gershwin ME, Chang C. Diagnosis and classification of idiopathic pulmonary fibrosis. Autoimmun Rev 2014; 13: 508-112.
13. Fletcher S, Jones GJ, Spinks, K, et al. The safety of new drugs treatment for idiopathic pulmonary fibrosis; Expert Opin Drug Saf 2016 Aug 17:1-7. [Epub ahead of print].
14. Canestaro WJ, Forrester SH, Raghu G, Ho L, Devine BE. Drug Treatment of Idiopathic Pulmonary Fibrosis: Systematic Review and Network Meta-Analysis. Chest 2016; 149: 756-66.
15. Prey S, Ezzedine K, Doussau A, et al. Imatinib mesylate in scleroderma-associated diffuse skin fibrosis: a phase II multicentre randomized double-blinded controlled trial. Br J Dermatol. 2012 ; 167: 1138-44.
16. Ene-Stroescu D, Ellman MH, Peterson CE. Topotecan and the development of scleroderma or a scleroderma-like illness. Arthritis Rheum 2005; 43: 840-50.
17. Rami G. Azrak et al. Therapeutic Synergy Between Irinotecan and 5-fluorouracil against Human Tumor Xenografts. Clin Cancer Res 2004; 10: 1121-9.

## Claims

1. Substance selected from the group comprising irinotecan, an irinotecan active metabolite, 5-fluoro-uracile, a pharmaceutical salt thereof, a prodrug thereof, a combination thereof and a mixture thereof, for use in the treatment of fibrosis.

2. Substance for use according to claim 1, wherein said pharmaceutical salt of irinotecan is selected from the group comprising hydrochloride and carboxylate sodium salt.

3. Substance for use according to claim 1, wherein said irinotecan active metabolite is 7-ethyl-10-hydroxy-camptothecin.

4. Substance for use according to claim 1, wherein said pharmaceutical salt of irinotecan active metabolite is carboxylate disodium salt.

5. Substance for use according to claim 1, wherein said pharmaceutical salt of 5-fluoro-uracile is sodium salt.

6. Substance for use according to anyone of the preceding claims, wherein said fibrosis is a scleroderma.

7. Substance for use according to claim 6, wherein said scleroderma is selected from the group comprising limited cutaneous scleroderma and diffuse cutaneous scleroderma.

8. Substance for use according to claim 6 or 7, wherein said substance decreases inflammation during the vascular injury stage of scleroderma.

9. Substance for use according to claim 6, wherein said fibrosis is a pulmonary fibrosis due to a scleroderma.

10. Substance according to anyone of claims 1 to 5, wherein said fibrosis is at least one fibrosis selected among idiopathic pulmonary fibrosis, retroperitoneal fibrosis and cystic fibrosis.

11. Substance for use according to anyone of the preceding claims, wherein said substance is a mixture of irinotecan and 5-fluoro-uracile, or a mixture of irinotecan active metabolite and 5-fluoro-uracile.

12. Substance for use according to anyone of the preceding claims, wherein said treatment comprises an administration of said substance by a route selected among oral route, buccal route, sublingual route, parenteral route, intravenous route, intramuscular route and subcutaneous route.


SEQUENCE LISTING

<110>  CENTRE HOSPITALIER UNIVERSITAIRE DE BORDEAUX
       UNIVERSITE DE BORDEAUX

<120>  Substance selected among Irinotecan, an irinotecan active
       metabolite, 5-fluoro-uracile, a pharmaceutical salt thereof and a
        mixture thereof, for use in the treatment of fibrosis

<130>  BNT221340EP00

<160>  12

<170>  PatentIn version 3.5

<210>  1
<211>  21
<212>  DNA
<213>  Artificial Sequence

<220>
<223>  primer sense

<400>  1
cgatggattc cagttcgagt a                                                21


<210>  2
<211>  20
<212>  DNA
<213>  Artificial Sequence

<220>
<223>  primer antisense

<400>  2
gtttacagga agcagacagg                                                  20


<210>  3
<211>  25
<212>  DNA
<213>  Artificial Sequence

<220>
<223>  primer sense

<400>  3
aggtgtaagc ggtggtggtt atgac                                            25


<210>  4
<211>  25
<212>  DNA
<213>  Artificial Sequence

<220>
<223>  primer antisense

<400>  4
ccggatacag gtttcgccag tagag                                            25

<210>  5
<211>  21
<212>  DNA
<213>  Artificial Sequence

<220>
<223>  primer sense

<400>  5
actgctgctg ctgctgttct g                                                21


<210>  6
<211>  25
<212>  DNA
<213>  Artificial Sequence

<220>
<223>  primer antisense

<400>  6
tgcttcatca ccttcagggt ttcag                                            25


<210>  7
<211>  26
<212>  DNA
<213>  Artificial Sequence

<220>
<223>  primer sense

<400>  7
tgcaattccg acctcgtcat cagggc                                           26


<210>  8
<211>  25
<212>  DNA
<213>  Artificial Sequence

<220>
<223>  primer antisense

<400>  8
agaaactcct cgctgcggtt gtggg                                            25


<210>  9
<211>  28
<212>  DNA
<213>  Artificial Sequence

<220>
<223>  primer sense

<400>  9
ttctcaaact gaagctcgca ctctcgcc                                         28


<210>  10
<211>  30
<212>  DNA

<213>  Artificial Sequence

<220>
<223>  primer antisense

<400>  10
tgtggagtga gtgttcaagt cttcggagtt                                        30


<210>  11
<211>  20
<212>  DNA
<213>  Artificial Sequence

<220>
<223>  primer sense

<400>  11
aatggctctg ggctctgtaa                                                   20


<210>  12
<211>  20
<212>  DNA
<213>  Artificial Sequence

<220>
<223>  primer antisense

<400>  12
tggtgatgat gccatgttct                                                   20